(19) Europäisches Patentamt
European Patent Office
Office européen des brevets

(11) **EP 3 451 276 A1**

(12) **EUROPEAN PATENT APPLICATION**
published in accordance with Art. 153(4) EPC

(43) Date of publication:
06.03.2019 Bulletin 2019/10

(51) Int Cl.:
*G06Q 50/22* $^{(2018.01)}$      *G06Q 10/10* $^{(2012.01)}$

(21) Application number: 16900520.4

(22) Date of filing: 13.06.2016

(86) International application number:
PCT/JP2016/067547

(87) International publication number:
WO 2017/187650 (02.11.2017 Gazette 2017/44)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 28.04.2016 JP 2016092035

(71) Applicant: Quintessensia LLC
Yokohama-shi, Kanagawa 222-0011 (JP)

(72) Inventors:
• MIYAKE, Masato
Kohoku-ku, Japan 222-0011 (JP)
• LIPOWSKI, Gerard
Kohoku-ku, Japan 222-0011 (JP)
• KAGIYAMA, Naoto
Kohoku-ku, Japan 222-0011 (JP)

(74) Representative: Hering, Hartmut
Patentanwälte
Berendt, Leyh & Hering
Innere Wiener Strasse 20
81667 München (DE)

(54) **HEALTH STATE EVALUATION SYSTEM, HEALTH STATE EVALUATION DEVICE, AND HEALTH STATE EVALUATION METHOD**

(57) Provided is a health state evaluation system which allows a layperson to know his/her rank in order to improve his/her health state. Through a principal component analysis using, as explanatory variables, health checkup values Dx which are principally blood values, three principal component variables with top three largest variances are obtained, the variables including an exercise habit variable C1, an energy intake habit variable C2, and a nutrition balance habit variable C3. The system includes: a data input section configured to input the health checkup values Dx; a computation section configured to calculate the respective habit variables C1 to C3, based on coefficients and formulae derived through the principal component analysis; and a result display section configured to display a rank of the subject among data of a large number of persons, obtained by the computation section in advance. The habit variables C1 to C3 include elements in 3 groups. The health state of the subject is evaluated by obtaining, according to 3 principal component variables C1 to C3, the rank of the subject among the data of the large number of persons.

Fig. 1

**Description**

TECHNICAL FIELD

The present invention relates to a health state evaluation system, a health state evaluation device, and a health state evaluation method. Specifically, the present invention relates to a health state evaluation system, a health state evaluation device, and a health state evaluation method for evaluating the health state of a subject by obtaining a rank of the subject among data, of a large number of persons, obtained in advance by using health checkup values (Dx) which are principally blood values.

BACKGROUND ART

Conventionally, an example of such a system has been disclosed in Non-Patent Document 1. According to this document, ranking for each of blood values or ranking by a principal component analysis for each of organs has been performed. The ranking of the health state requires professional interpretation, and therefore is not suitable for a layperson to use for improvement of his/her health state.

CITATION LIST

[NON-PATENT DOCUMENTS]

[NON-PATENT DOCUMENT 1] Japan Society of Ningen Dock/National Federation of Health Insurance Societies, "Reference Range for basic tests of new medical checkup, MEGA Study of 1.5 million subjects by the Japan Society of Ningen Dock and the National Federation of Health Insurance Societies", [online], April 2014, the Internet <URL:http://www.ningen-dock.jp/wp/wp-content/uploads/2013/09/%E3%83%97%E3%83%AC%E3%82%B9%E3%83%AA%E3%83%A A%E3%83%BC%E3%82%B9%E7%94%A8PDF%EF%BC%88140409%E5%B7%AE%E3%8 1%97%E6%9B%BF%E3%81%88%EF%BC%89.pdf>

SUMMARY OF THE INVENTION

PROBLEMS TO BE SOLVED BY THE INVENTION

In view of the conventional circumstances described above, an object of the present invention is to provide a health state evaluation system which allows a layperson to know his/her rank in order to improve his/her health state.

SOLUTION TO THE PROBLEMS

In order to attain the above object, a health state evaluation system according to the present invention is configured to evaluate a health state of a subject by obtaining a rank of the subject among data of a large number of persons, the data having been obtained in advance by using health checkup values (Dx) which are principally blood values. Through a principal component analysis using the health checkup values (Dx) as explanatory variables, principal component variables with top three largest variances are obtained, the variables including an energy intake habit variable (C1), a nutrition balance habit variable (C2), and an exercise habit variable (C3). This system includes: a data input section configured to input the health checkup values (Dx); a computation section configured to calculate the respective habit variables (C1 to C3), based on coefficients and formulae derived through the principal component analysis; a storage section configured to store therein the habit variables (C1 to C3) which have been obtained from a large number of persons in advance; and a result display section configured to display a rank of the subject regarding each of the habit variables (C1 to C3) among the habit variables (C1 to C3) of the large number of persons, the rank having been obtained by the computation section. The health state of the subject is evaluated by obtaining, according to the habit variables (C1 to C3), the rank of the subject among the data of the large number of persons, the data having been obtained in advance.

In this system, Ax is a weight coefficient of each Dx, which is derived through the principal component analysis,

$$\text{energy intake habit variable } C1 = \Sigma(A1,n1*D1,n1)$$

2

(D1,n1 includes one or some of minimal blood pressure, AST, ALT, and blood glucose; n1 is a natural number, $\Sigma$ 1 to n1),

$$\text{nutrition balance habit variable C2} = \Sigma(A2,n2*D2,n2) + \Sigma(A1,n1*D1,n1)$$

(D2,n2 includes one or some of LDL, HDL, $\gamma$GTP, and neutral fats; n2 and n1 are natural numbers, $\Sigma$ 1 to n2, $\Sigma$ 1 to n1), and

$$\text{exercise habit variable C3} = \text{A3(intrinsic coefficient of maximal blood pressure)}*\text{D3(maximal blood pressure)} + \Sigma(A1,n1*D1,n1)$$

(n1 is a natural number, $\Sigma$ 1 to n1).

With the above configuration, the inventors have found that the principal component variables with top three largest variances correspond to the exercise habit variable (C1), the energy intake habit variable (C2), and the nutrition balance habit variable (C3), and are applicable as indices for lifestyle improvement.

Therefore, by only using the health checkup values (Dx) which are principally blood values, the subject can know the exercise habit variable (C1), the energy intake habit variable (C2), and the nutrition balance habit variable (C3). Thus, even a layperson can easily attempt to improve his/her health state.

Preferably, the result display section displays, together with the habit variables (C1) to (C3), the number of examinees and a prevalence for each variable.

In the above case, preferably, the result display section plots markers as a result of the subject on a graph of the prevalence at positions corresponding to values of the habit variables (C1) to (C3), thereby displaying the prevalence.

Preferably, the result display section is represented as three types of two-dimensional graphs obtained by extracting two components from any of the habit variables (C1) to (C3), and/or a three-dimensional graph having the three variables as components thereof.

Preferably, the result display section displays, on the graph, a plurality of measurement processes in time sequence.

Preferably, the result display section identifies and displays the values of the habit variables (C1) to (C3) in a plurality of stages.

Preferably, the energy intake habit variable C1, the nutrition balance habit variable C2, and the exercise habit variable C3 are represented by formulae as follows:

$$\text{the energy intake habit variable C1} = \text{A1}*\text{D1(minimal blood pressure)};$$

$$\text{the nutrition balance habit variable C2} = \text{A2}*\text{D2(neutral fats)} + \text{A1}*\text{D1(minimal blood pressure)};$$

$$\text{the exercise habit variable C3} = \text{A3}*\text{D3(maximal blood pressure)} + \text{A1}*\text{D1(minimal blood pressure)}.$$

Preferably, the energy intake habit variable C1, the nutrition balance habit variable C2, and the exercise habit variable C3 are represented by formulae as follows:

$$\text{the energy intake habit variable C1} = \text{A11}*\text{D11(minimal blood pressure)} + \text{A12}*\text{D12(AST)} + \text{A13}*\text{D13(ALT)} + \text{A14}*\text{D14(blood glucose)};$$

$$\text{the nutrition balance habit variable } C2 = \{A21*D21(\text{neutral fats})+A22*D22(\text{LDL})+A23*D23(\text{HDL})+A24*D24(\gamma GTP)\} + \{A11*D11(\text{minimal blood pressure})+A12*D12(\text{AST})+A13*D13(\text{ALT})+A14*D14(\text{blood glucose })\};$$

and

$$\text{the exercise habit variable } C3 = A3*D3(\text{maximal blood pressure}) + \{A11*D11(\text{minimal blood pressure})+A12*D12(\text{AST})+A13*D13(\text{ALT})+A14*D14(\text{blood glucose })\}.$$

In order to attain the above object, a health state evaluation device according to the present invention is configured to evaluate a health state of a subject by obtaining a rank of the subject among data of a large number of persons, the data having been obtained in advance by using health checkup values (Dx) which are principally blood values. Through a principal component analysis using the health checkup values (Dx) as explanatory variables, principal component variables with top three largest variances are obtained, the variables including an energy intake habit variable (C1), a nutrition balance habit variable (C2), and an exercise habit variable (C3). This device includes: a data input section configured to input the health checkup values (Dx); a computation section configured to calculate the respective habit variables (C1 to C3), based on coefficients and formulae derived through the principal component analysis; a storage section configured to store therein the habit variables (C1 to C3) which have been obtained from a large number of persons in advance; and a result display section configured to display a rank of the subject regarding each of the habit variables (C1 to C3) among the habit variables (C1 to C3) of the large number of persons, the rank having been obtained by the computation section. The health state of the subject is evaluated by obtaining, according to the habit variables (C1 to C3), the rank of the subject among the data of the large number of persons, the data having been obtained in advance. In this device, Ax, A1 to A3, C1 to C3, D1 to D3, n1, and n2 are identical to those described above.

In order to attain the above object, a health state evaluation method according to the present invention is configured to evaluate a health state of a subject by obtaining a rank of the subject among data of a large number of persons, the data having been obtained in advance by using health checkup values (Dx) which are principally blood values. Through a principal component analysis using the health checkup values (Dx) as explanatory variables, principal component variables with top three largest variances are obtained, the variables including an energy intake habit variable (C1), a nutrition balance habit variable (C2), and an exercise habit variable (C3). This method includes: inputting the health checkup values (Dx); calculating the respective habit variables (C1 to C3), based on coefficients and formulae derived through the principal component analysis, and on the inputted variables; storing, in a storage section, the habit variables (C1 to C3) which have been obtained from a large number of persons in advance; and displaying, as a graph, a rank of the subject regarding each of the habit variables (C1 to C3) among the habit variables (C1 to C3) of the large number of persons, the rank having been obtained by the computation section. The health state of the subject is evaluated by obtaining, according to the habit variables (C1 to C3), the rank of the subject among the data of the large number of persons, the data having been obtained in advance. In this method, Ax, A1 to A3, C1 to C3, D1 to D3, n1, and n2 are identical to those described above.

In order to attain the above object, a health state evaluation system according to the present invention is configured to evaluate a health state of a subject by obtaining a rank of the subject among data of a large number of persons, the data having been obtained in advance by using health checkup values (Dx) which are principally blood values. Through a principal component analysis using the health checkup values (Dx) as explanatory variables, a nutrition balance habit variable (C2) and an exercise habit variable (C3) are obtained among principal component variables with top three largest variances. This system includes: a data input section configured to input the health checkup values (Dx); a computation section configured to calculate the respective habit variables (C2, C3), based on coefficients and formulae derived through the principal component analysis; a storage section configured to store therein the habit variables (C2, C3) which have been obtained from a large number of persons in advance; and a result display section configured to display a rank of the subject regarding each of the habit variables (C2, C3) among the habit variables (C2, C3) of the large number of persons, the rank having been obtained by the computation section. The health state of the subject is evaluated by obtaining, according to the habit variables (C2, C3), the rank of the subject among the data of the large number of persons, the data having been obtained in advance.

In this system, Ax is a weight coefficient of each Dx, which is derived through the principal component analysis,

$$\text{nutrition balance habit variable } C2 = \Sigma(A2,n2*D2,n2) + \Sigma(A1,n1*D1,n1)$$

(D1,n1 includes one or some of minimal blood pressure, AST, ALT, and blood glucose; n1 is a natural number, $\Sigma$ 1 to n1)
(D2,n2 includes one or some of LDL, HDL, $\gamma$GTP, and neutral fats; n2 is a natural number, $\Sigma$ 1 to n2), and

$$\text{exercise habit variable } C3 = A3(\text{intrinsic coefficient of maximal blood pressure})*D3(\text{maximal blood pressure}) + \Sigma(A1,n1*D1,n1)$$

(n1 is a natural number, $\Sigma$ 1 to n1).

ADVANTAGEOUS EFFECTS OF THE INVENTION

According to the features of the health state evaluation system, the health state evaluation device, and the health state evaluation method of the present invention, a layperson is allowable to know his/her rank which is useful for health state improvement.

Other objects, configurations, and effects of the present invention will become apparent from the following description of embodiments of the present invention.

BRIEF DESCRIPTION OF THE DRAWINGS

[FIG. 1] FIG. 1 is a block diagram showing a health state evaluation system (device) according to the present invention.
[FIG. 2] FIG. 2 illustrates graphs each showing a relationship between health checkup values (Dx) and a mutual relationship of habit variables (components 1 to 3, C1 to C3) as three principal component valuables, in which (a) shows the relationship with C1 and C2, (b) shows the relationship with C2 and C3, and (c) shows the relationship with C1 and C3.
[FIG. 3] FIG. 3 illustrates graphs each showing percentages of diseases of persons in higher ranks regarding the values of the habit variables (i.e., those having life habits in red (Pcx) zones).
[FIG. 4] FIG. 4 illustrates graphs each showing a relationship between the habit variable (C1 to C3) and a prevalence, in which an upper stage shows a distribution of the habit variable (C1 to C3), a lower stage shows a prevalence regarding the habit variable (C1 to C3), and (a), (b), and (c) correspond to energy intake C1, nutrition balance C2, and an amount of exercise C3, respectively.
[FIG. 5] FIG. 5 illustrates graphs showing a display example of this system, each graph showing the health state of a subject by integrating each habit variable (C1 to C3) with the prevalence, in which (a), (b), and (c) correspond to energy intake C1, nutrition balance C2, and an amount of exercise C3, respectively.
[FIG. 6] FIG. 6 illustrates zone graphs showing another display example of this system, in which (a), (b), and (c) correspond to energy intake C1, nutrition balance C2, and an amount of exercise C3, respectively.
[FIG. 7] FIG. 7 illustrates subject monitoring states by using graphs of the habit variables (C1 to C3) of this system.
[FIG. 8] FIG. 8 illustrates other subject monitoring states by using graphs of the habit variables (C1 to C3) of this system.
[FIG. 9] FIG. 9 illustrates graphs each showing a relationship between the habit variable (C1 to C3) and a prevalence, obtained through evaluation of the present invention using only three health checkup values (Dx).
[FIG. 10] FIG. 10 is a graph showing the health state of a subject by assigning the habit variables (C1 to C3) to different axes of a three-dimensional graph.

DESCRIPTION OF EMBODIMENTS

Next, the present invention will be described in more detail with reference to the accompanying drawings as appropriate.

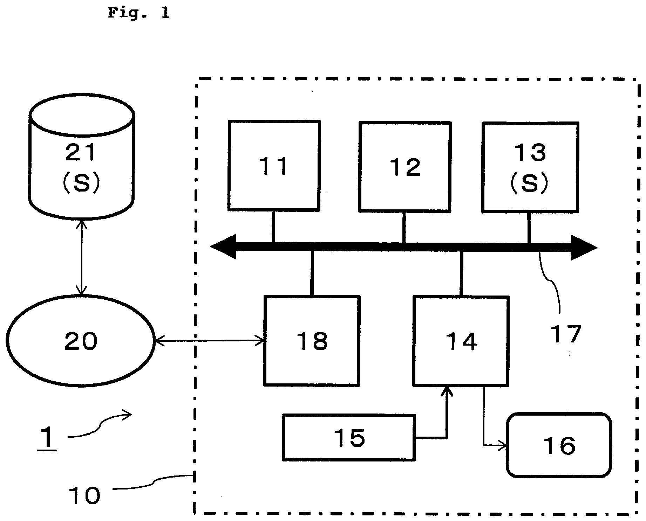

FIG. 1 is a block diagram showing a health state evaluation system (device) 1 according to the present invention. A PC 10 includes: a computation section 11 which performs computation processing and creates a display image; a temporary storage section 12; a storage section 13; an input/output IF 14; a communication IF 18; and an internal bus 17 including a data bus and an address bus for connecting these components. An input section 15 such as a keyboard and a mouse used as a data input section, and a display section 16 such as a display and a printer used as a result display section, are connected to the input/output IF 14. The communication IF 18 is connected to an external storage 21 and other PCs (not shown) via a network 20 established by ICP/IP or the like.

The PC 10 is used for data input, principal component analysis by arithmetic processing, and data display. Habit variables (C1 to C3) obtained in advance from a large number of persons are stored in the storage section 13 in the PC 10, and are stored as a storage section in the external storage 21. As for display of arithmetic results, the display section 16 is caused to display graphs and input/output screens described later.

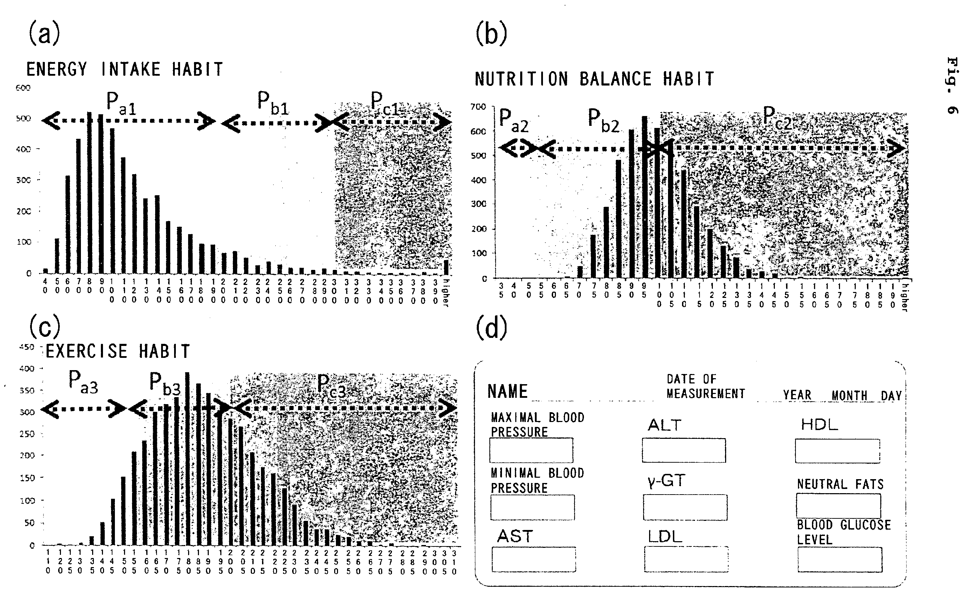

As for health checkup values (Dx) which are principally blood values, there are 9 numerical values displayed on an input screen shown in FIG. 6(d), and the adequate values and details of the respective values are as follows.

AST, 10-40 IU/l: AST is an enzyme found primarily in the liver. If tissues are impaired, the AST level in blood increases. An extremely high AST level is a sign of various types of liver damage.

AST, 5-45 IU/l: ALT is an enzyme found primarily in the liver. If tissues are impaired, the ALT level in blood increases. An extremely high ALT level is a sign of various types of liver damage.

$\gamma$-GT, 80 IU/l or lower (male), 30 IU/l or lower (female): $\gamma$-GT is an enzyme spreading in the liver, etc., and increases due to cholestasis, alcohol, drugs, etc. If cholestasis is caused by hepatitis, obstructive jaundice, gallstone, or the like, the measured value increases. In particular, $\gamma$-GT sensitively reacts with alcohol, and sometimes increases due to drugs such as analgesic.

LDL: LDL is calculated by subtracting HDL from total cholesterol.

HDL, 40-70 mg/dl (male), 45-75 mg/dl (female): HDL (High Density Lipoprotein) cholesterol delivers cholesterol that may cause arteriosclerosis from peripheral arteries to the liver, and therefore is also called "good cholesterol". HDL cholesterol not higher than 40 mg/dl is highly likely to cause arteriosclerosis. Although HDL cholesterol cannot be increased directly through food intake, body fat reduction and moderate exercise are effective.

Neutral fats, 30-149 mg/dl: neutral fats are used as an energy source. However, excessive neutral fats are accumulated under the skin and/or in the liver, and cause obesity and/or fatty liver disease. In addition, excessive neutral fats accelerate arteriosclerosis.

Blood glucose level (fasting blood glucose level), 70-109 mg/dl: A blood glucose test is a test for measuring glucose concentration in blood. Glucose is used and metabolized as an energy source in a living body. The blood glucose level varies with diet, etc., but the variable range is within a fixed range. Diencephalon, autonomic nerve, and hormones such as insulin are deeply involved in control of the blood glucose level. Generally, diagnosis of glucose metabolism is performed with a fasting blood glucose level in the morning. Although it is difficult to perform accurate diagnosis with a casual blood glucose level (or after-meal blood glucose level) other than a fasting blood glucose level, blood glucose levels of normal persons hardly exceed 140 mg/dl even after meals.

Maximal blood pressure: "maximal blood pressure" is a pressure when the muscle of the heart is contracted maximally so that the heart pumps blood, and is also called "systolic blood pressure".

Minimal blood pressure: "minimal blood pressure" is a pressure when the muscle of the heart is expanded maximally, and is also called "diastolic blood pressure".

Adequate values of blood pressure are shown in the following Table 1.

[Table 1]

| List of blood pressure normal values | Maximal blood pressure | Minimal blood pressure | When measured at home |
|---|---|---|---|
| Optimal blood pressure | up to 119 | up to 79 | Lower than 125/80 |
| Normal blood pressure | 120~ | 80~84 | |
| High normal blood pressure | 130~ | 85~89 | |
| High blood pressure (stage I) | 140~ | 90~99 | 135/85 or higher |
| High blood pressure (stage II) | 160~ | 100~109 | |
| High blood pressure (stage III) | 180 or higher | 110 or higher | |
| unit: mmHg | | | |

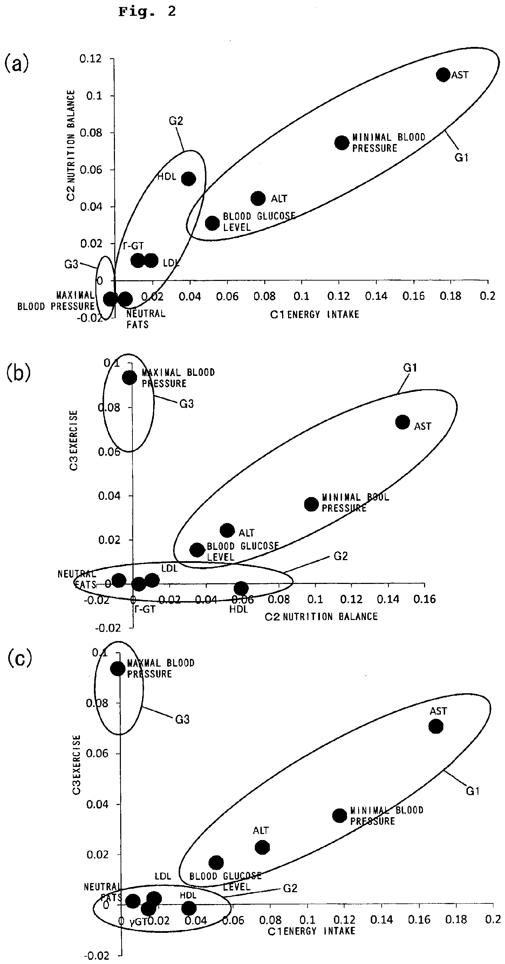

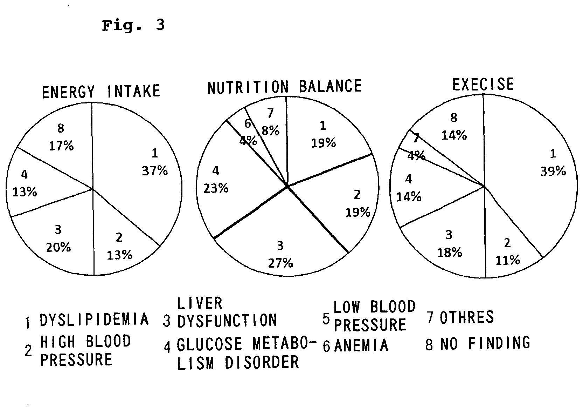

With reference to FIG. 2, a description will be given of a relationship between each of the above-described 9 health checkup values (Dx), and a mutual relationship of habit variables (components 1 to 3; hereinafter referred to as "C1 to C3") which are 3 principal component valuables analyzed by using data of about 5,000 persons (4,717 subjects, the same applies hereinafter). The 9 health checkup values (Dx) are selected through trial and error so that variances of C1 to C3 become great. FIGS. 2(a), 2(b), and 2(c) are graphs showing scattergrams of principle component load amounts regarding the relationship between C1 and C2, the relationship between C2 and C3, and the relationship between C1 and C3, respectively. In each graph, plotted points represent coefficients of the health checkup values (Dx) regarding C1 to C3. In each of the scattergrams, blood-related components are separated into three groups G1 to G3. In addition, FIG. 3 illustrates graphs each showing the percentages of diseases of persons in higher ranks regarding the values of the habit variable (C1 to C3) (i.e., those having life habits in red (Pcx) zones shown in FIG. 6(d)).

The aforementioned facts will be analyzed hereinafter. In the graph having C2 and C3 axes shown in FIG. 2(b), G2 and G3 are on the C2 and C3 axes, respectively, but G1 is neither on the C2 axis nor on the C3 axis. In the graph having C1 and C2 axes shown in FIG. 2(a), G3 is approximately zero, and each of G1 and G2 is neither on the C1 axis nor on the C2 axis. In the graph having C1 and C3 axes shown in FIG. 2(c), G2 is approximately zero, G3 has values on the C3 axis, and G1 is neither on the C1 axis nor on the C3 axis. Thus, C1 is correlated with G1 but is independent from G2 and G3, C2 is correlated with G1 and G2, and C3 is correlated with G1 and G3.

Meanwhile, consideration is given on the relationship between C1 to C3 and life habits, from the viewpoint of biological chemistry. The group G1 composing C1 includes blood glucose that can be controlled by the energy intake amount. Other elements in G1 are considered to be in the same group because they vary in association with blood glucose. Therefore, C1 is a parameter necessary for calculation of an energy intake habit score.

The group G2 composing C2 includes neutral fats that can be controlled by nutrition balance. HDL and the like in the same group G2 vary in association with neutral fats. Since the group G1 also depends on C2, the elements in G1 as well as G2 are used as parameters for score calculation. The group G3 composing C3 includes only systolic blood pressure, which is a factor that can be controlled by exercise. For calculation of an exercise habit score, G1 that depends on C3 is also used. These findings reveal that the elements in G1 should be used for C1 that reflects energy intake, the elements of G2 and G1 should be used for C2 that reflects nutrition balance, and the elements of G3 and G1 should be used for C3 that reflects exercise. Furthermore, the above findings reveal that the respective habit variables (C1 to C3) are represented as in claim 8, the health checkup values Dx may be the values regarding three components as in claim 7 described later, and general formulae are expressed as in claim 1.

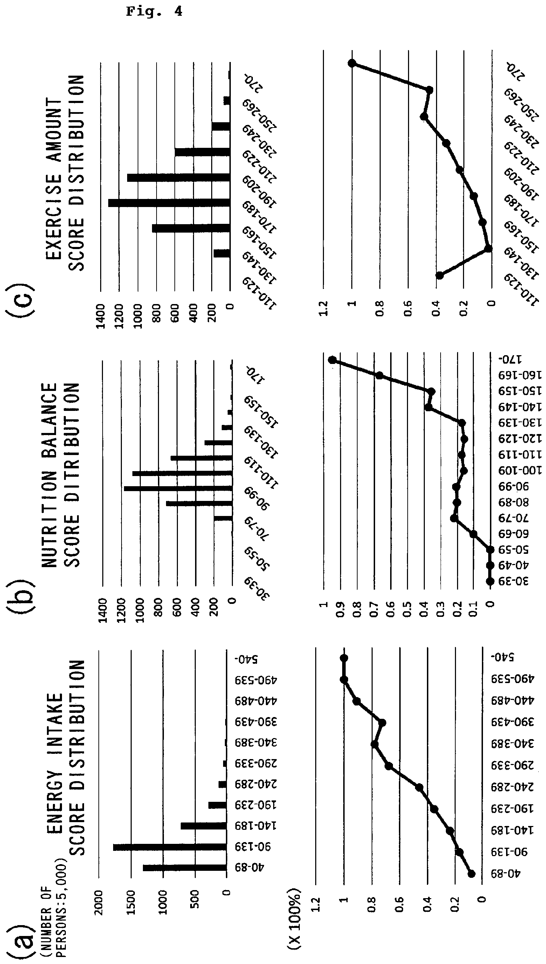

Here, verification was performed on the relationship between prevalence of lifestyle-related diseases and each of the energy intake habit score C1, the nutrition balance habit score C2, and the exercise habit score C3. The 3 habit variables (C1 to C3) of 4,717 persons were obtained based on anonymized individual blood-related component matrices of 4,717 persons obtained using, as elements, blood pressure (during systole), blood pressure (during diastole), ALT, AST, $\gamma$-GT, LDL, HLD, neutral fats, and blood glucose, and on inner products of eigenvectors of the respective principal components. Next, frequency distributions of the 3 habit variables (C1 to C3) of 4,717 persons were created, and the percentage of sick persons included in each score rank was obtained. As the result, graphs shown in FIG. 4 were obtained, each showing the relationship between the habit variable (C1 to C3) and the prevalence. Since a positive correlation was observed between the score and the prevalence for each of the 3 habit variables (C1 to C3), it was confirmed that these scores were useful for guidance of lifestyle improvement.

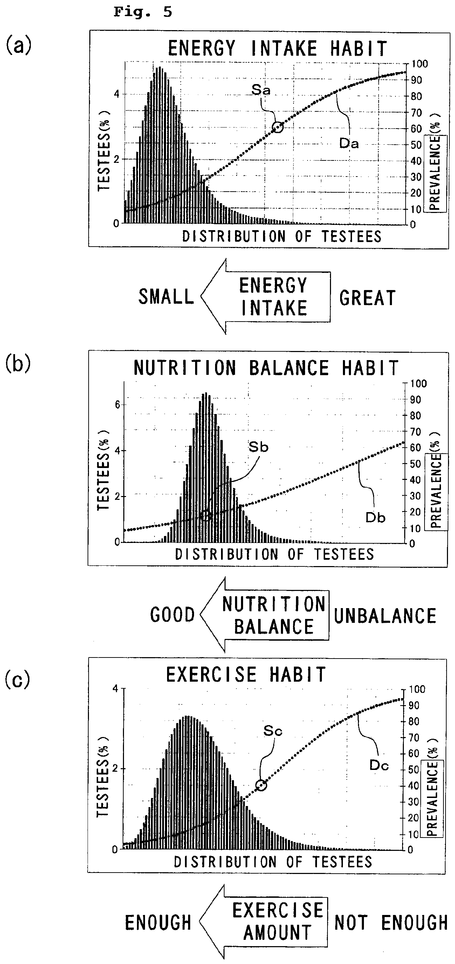

In practical use, as shown in FIG. 5, the distributions of the habit variables (C1 to C3) and the graphs of prevalence Da, Db, and Dc may be integrated with each other, respectively, for convenience. In this case, the state of a subject may be indicated by signs Sa, Sb, and Sc on the prevalence graphs Da, Db, and Dc, respectively. Preferably, the prevalence at each sign may be represented as a numerical value.

In practical use, zone graphs may be used as shown in FIG. 6.

As shown in FIG. 6, blue shows a zone indicated by a sign Pax, yellow shows a zone indicated by a sign Pbx, and red shows a zone indicated by a sign Pcx (x = 1 to 3). Each life habit is appropriate in order of blue > yellow > red. Attention is paid to the most inappropriate habit among the three life habits C1 to C3.

Blue zone: although the risk of lifestyle-related diseases is low, attention needs to be paid to anemia and hypotension .

Yellow zone: the risk of lifestyle-related diseases is likely to increase with aging.

Red zone: 80% of persons having the life habit in this zone suffer from lifestyle-related diseases. Appropriate lifestyle improvement is needed under guidance of a specialist such as a nutritionist.

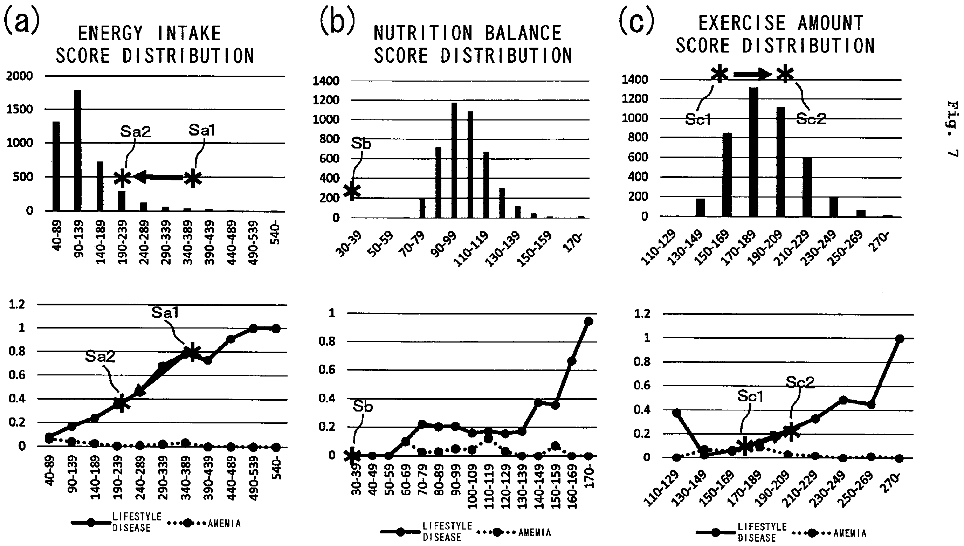

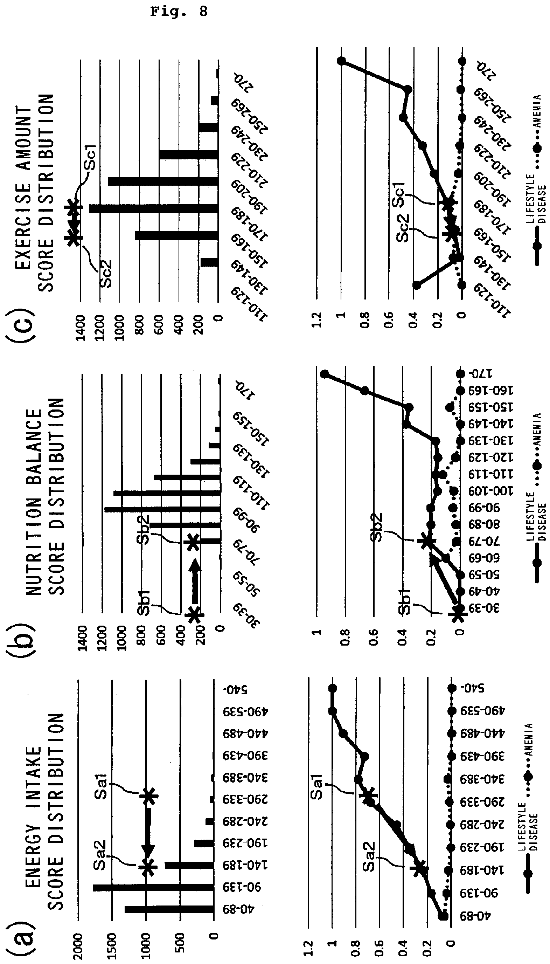

FIGS. 7 and 8 show examples of application to subjects. FIGS. 7 and 8 each show habit variable values Sa1, Sb1, and Sc1 of subjects before lifestyle guidance, and habit variable values Sa2, Sb2, and Sc2 of the subjects after lifestyle guidance. A sign Sb indicates a habit variable value that has not been changed. As the result of the lifestyle guidance, each subject commented that the graph allows the subject to easily comprehend the result of the guidance, and therefore serves as a source of motivation for lifestyle improvement.

Score calculation based on less data: since the 9 blood-related components were separated into 3 groups, one blood-related component data was arbitrarily selected from each group to be used for score calculation.

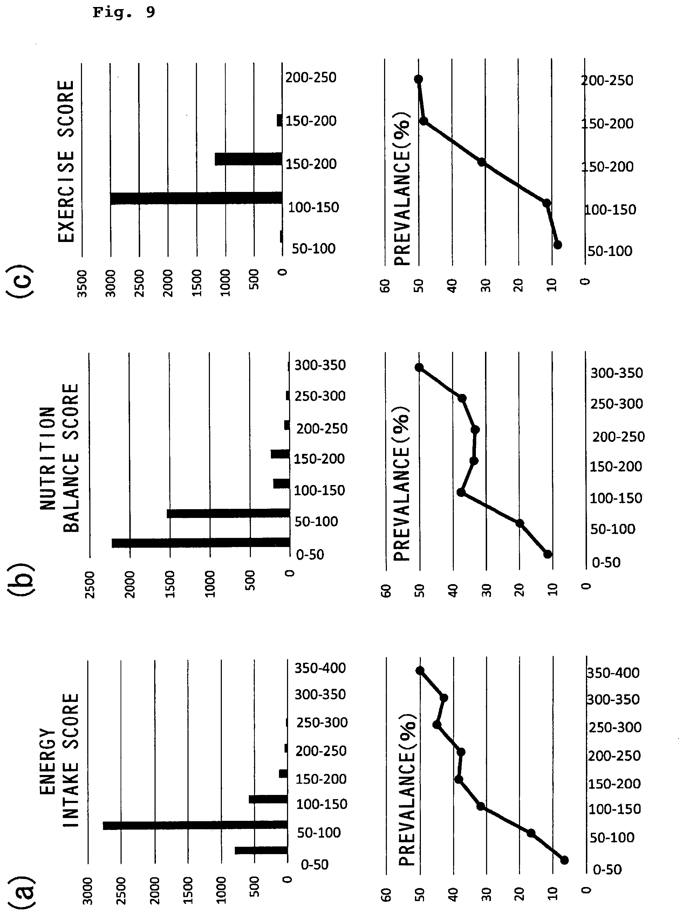

Incidentally, maximal blood pressure (during systole), minimal blood pressure (during diastole), and blood lipid (neutral fats) are parameters that belong to different groups G1 to G3, respectively. Among the previously obtained load amount eigenvalues of the 9 blood-related components, the eigenvalues of the above 3 parameters were used to obtain new eigenvectors regarding the energy intake habit, the nutrition balance habit, and the exercise habit. Based on the anonymized individual blood-related component matrixes [maximal blood pressure (during systole), minimal blood pressure (during diastole), and blood lipid] of 4,717 persons and on inner products of the eigenvectors, scores of 4,717 individuals were newly obtained to create frequency distribution diagrams. In addition, prevalence in each rank was calculated and graphed as shown in FIG. 9. A positive correlation was observed between the prevalence (%) and each life habit score calculated based on the 3 parameters including maximal blood pressure (during systole), minimal blood pressure (during diastole), and blood lipid. This fact revealed that the 3 blood-related component data provide the scores that are useful for lifestyle guidance. In this case, data that requires collection of blood is only blood lipid, and thus significant reduction in the test cost can be expected. Elements other than the three elements may be selected according to the aforementioned general formulae.

Lastly, other possible embodiments of the present invention will be described. The same members and elements as those of the above embodiment are denoted by the same reference signs. In addition, the respective embodiments can be mutually combined and executed.

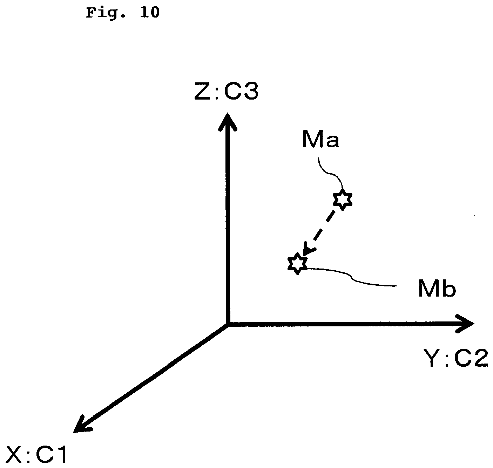

In the above-described embodiment, the respective habit variables (C1 to C3) are assigned to different graphs. However, as shown in FIG. 10, the respective habit variables (C1 to C3) may be assigned to different axes of a three-dimensional graph, and the state of each subject may be represented by three-dimensional coordinates with signs Ma, Mb, etc. At this time, if the zone graphs shown in FIG. 6 are applied to the three-dimensional coordinates, the zone graphs allow the subject to easily comprehend his/her state, and therefore are convenient as indices for lifestyle improvement.

In the above-described embodiment, the 3 habit variables (C1 to C3) are used. However, while the energy intake habit variable C1 includes only the elements in G1, the nutrition balance habit variable C2 and the exercise habit variable C3 include the elements in G1 and G2 and the elements in G1 and G3, respectively. Therefore, it is conceivable that diagnosis regarding G1 has already been incorporated in each of the nutrition balance habit variable C2 and the exercise habit variable C3, and therefore, only C2 and C3 may be used as indices for lifestyle improvement. In this case, general formulae as in claim 11 are adopted. In this case, as for graphs for diagnosis, two graphs or a two-dimensional plane graph are/is used. The configuration in practicing the present invention can use the elements described in claims 1 to 10, and the elements are disclosed in the present specification. However, it is needless to say that using 3 elements as indices for lifestyle improvement is advantageous from the viewpoint of the number of improvement points.

The gist of the present invention resides in that the health checkup values (Dx) in the group G1 are used as the energy intake habit variable C1, the health checkup values (Dx) in the groups G1 and G2 are used as the nutrition balance habit variable C2, and the health checkup values (Dx) in the groups G1 and G3 are used as the exercise habit variable C3.

Although the principal component analysis is used for obtaining the habit variables C1 to C3 and the coefficients of the above-described general formulae, another mathematical technique may be used. A method using the other mathematical technique is also within the scope of the present invention.

INDUSTRIAL APPLICABILITY

The present invention can be used as a health state evaluation system, a health state evaluation device, and a health state evaluation method in which the health state of a subject is evaluated by a method of scoring the health state based on his/her life habits.

DESCRIPTION OF THE REFERENCE CHARACTERS

1      health state evaluation system (device)
10     PC (Personal Computer)
11     computation section
12     temporary storage section
13     storage section
14     input/output IF (interface)
17     internal bus
18     communication IF (interface)
20     network
21     external storage

**Claims**

1. A health state evaluation system configured to evaluate a health state of a subject by obtaining a rank of the subject among data of a large number of persons, the data having been obtained in advance by using health checkup values (Dx) which are principally blood values, wherein
through a principal component analysis using the health checkup values (Dx) as explanatory variables, principal component variables with top three largest variances are obtained, the variables including an energy intake habit variable (C1), a nutrition balance habit variable (C2), and an exercise habit variable (C3),
the health state evaluation system comprising:

a data input section configured to input the health checkup values (Dx);
a computation section configured to calculate the respective habit variables (C1 to C3), based on coefficients and formulae derived through the principal component analysis;
a storage section configured to store therein the habit variables (C1 to C3) which have been obtained from a large number of persons in advance; and
a result display section configured to display a rank of the subject regarding each of the habit variables (C1 to C3) among the habit variables (C1 to C3) of the large number of persons, the rank having been obtained by the computation section, wherein
the health state of the subject is evaluated by obtaining, according to the habit variables (C1 to C3), the rank of the subject among the data of the large number of persons, the data having been obtained in advance, where
Ax is a weight coefficient of each Dx, which is derived through the principal component analysis,

$$\text{energy intake habit variable } C1 = \Sigma(A1,n1 * D1,n1)$$

(D1,n1 includes one or more of minimal blood pressure, AST, ALT, and blood glucose; n1 is a natural number, $\Sigma$ 1 to n1),

$$\text{nutrition balance habit variable } C2 = \Sigma(A2,n2 * D2,n2) + \Sigma(A1,n1 * D1,n1)$$

(D2,n2 includes one or more of LDL, HDL, γGTP, and neutral fats; n2 and n1 are natural numbers, $\Sigma$ 1 to n2, $\Sigma$ 1 to n1), and

$$\text{exercise habit variable } C3 = A3(\text{intrinsic coefficient of maximal blood pressure}) * D3(\text{maximal blood pressure}) + \Sigma(A1,n1 * D1,n1)$$

(n1 is a natural number, $\Sigma$ 1 to n1).

2. The health state evaluation system according to claim 1, wherein the result display section displays, together with the habit variables (C1) to (C3), the number of examinees and a prevalence for each variable.

3. The health state evaluation system according to claim 2, wherein the result display section plots markers as a result of the subject on a graph of the prevalence at positions corresponding to values of the habit variables (C1) to (C3), thereby displaying the prevalence.

4. The health state evaluation system according to claim 1, wherein the result display section is represented as three types of two-dimensional graphs obtained by extracting two components from any of the habit variables (C1) to (C3), and/or a three-dimensional graph having the three variables as components thereof.

5. The health state evaluation system according to any one of claims 2 to 4, wherein the result display section displays, on the graph, a plurality of measurement processes in time sequence.

6. The health state evaluation system according to any one of claims 2 to 4, wherein the result display section identifies and displays the values of the habit variables (C1) to (C3) in a plurality of stages.

7. The health state evaluation system according to any one of claims 2 to 4, wherein the energy intake habit variable C1, the nutrition balance habit variable C2, and the exercise habit variable C3 are represented by formulae as follows:

$$\text{the energy intake habit variable } C1 = A1*D1(\text{minimal blood pressure});$$

$$\text{the nutrition balance habit variable } C2 = A2*D2(\text{neutral fats}) + A1*D1(\text{minimal blood pressure});$$

$$\text{the exercise habit variable } C3 = A3*D3(\text{maximal blood pressure}) + A1*D1(\text{minimal blood pressure}).$$

8. The health state evaluation system according to any one of claims 2 to 4, wherein the energy intake habit variable C1, the nutrition balance habit variable C2, and the exercise habit variable C3 are represented by formulae as follows:

$$\text{the energy intake habit variable } C1 = A11*D11(\text{minimal blood pressure}) + A12*D12(\text{AST}) + A13*D13(\text{ALT}) + A14*D14(\text{blood glucose});$$

$$\text{the nutrition balance habit variable } C2 = \{A21*D21(\text{neutral fats})+A22*D22(\text{LDL})+A23*D23(\text{HDL})+A24*D24(\gamma\text{GTP})\} + \{A11*D11(\text{minimal blood pressure})+A12*D12(\text{AST})+A13*D13(\text{ALT})+A14*D14(\text{blood glucose})\};$$

$$\text{the exercise habit variable } C3 = A3*D3(\text{maximal blood pressure}) + \{A11*D11(\text{minimal blood pressure})+A12*D12(\text{AST})+A13*D13(\text{ALT})+A14*D14(\text{blood glucose})\}.$$

9. A health state evaluation device configured to evaluate a health state of a subject by obtaining a rank of the subject among data of a large number of persons, the data having been obtained in advance by using health checkup values (Dx) which are principally blood values, wherein
through a principal component analysis using the health checkup values (Dx) as explanatory variables, principal component variables with top three largest variances are obtained, the variables including an energy intake habit variable (C1), a nutrition balance habit variable (C2), and an exercise habit variable (C3),
the health state evaluation device comprising:

a data input section configured to input the health checkup values (Dx);
a computation section configured to calculate the respective habit variables (C1 to C3), based on coefficients and formulae derived through the principal component analysis;
a storage section configured to store therein the habit variables (C1 to C3) which have been obtained from a large number of persons in advance; and
a result display section configured to display a rank of the subject regarding each of the habit variables (C1 to C3) among the habit variables (C1 to C3) of the large number of persons, the rank having been obtained by the computation section, wherein
the health state of the subject is evaluated by obtaining, according to the habit variables (C1 to C3), the rank of the subject among the data of the large number of persons, the data having been obtained in advance, where

Ax, A1 to A3, C1 to C3, D1 to D3, n1, and n2 are identical to those of claim 1.

10. A health state evaluation method for evaluating a health state of a subject by obtaining a rank of the subject among data of a large number of persons, the data having been obtained in advance by using health checkup values (Dx) which are principally blood values, wherein
through a principal component analysis using the health checkup values (Dx) as explanatory variables, principal component variables with top three largest variances are obtained, the variables including an energy intake habit variable (C1), a nutrition balance habit variable (C2), and an exercise habit variable (C3), the health state evaluation method comprising:

inputting the health checkup values (Dx);
calculating the respective habit variables (C1 to C3), based on coefficients and formulae derived through the principal component analysis, and on the inputted variables;
storing, in a storage section, the habit variables (C1 to C3) which have been obtained from a large number of persons in advance; and
displaying, as a graph, a rank of the subject regarding each of the habit variables (C1 to C3) among the habit variables (C1 to C3) of the large number of persons, the rank having been obtained by the computation section, wherein
the health state of the subject is evaluated by obtaining, according to the habit variables (C1 to C3), the rank of the subject among the data of the large number of persons, the data having been obtained in advance, where
Ax, A1 to A3, C1 to C3, D1 to D3, n1, and n2 are identical to those of claim 1.

11. A health state evaluation system configured to evaluate a health state of a subject by obtaining a rank of the subject among data of a large number of persons, the data having been obtained in advance by using health checkup values (Dx) which are principally blood values, wherein
through a principal component analysis using the health checkup values (Dx) as explanatory variables, a nutrition balance habit variable (C2) and an exercise habit variable (C3) are obtained among principal component variables with top three largest variances,
the health state evaluation system comprising:

a data input section configured to input the health checkup values (Dx);
a computation section configured to calculate the respective habit variables (C2, C3), based on coefficients and formulae derived through the principal component analysis;
a storage section configured to store therein the habit variables (C2, C3) which have been obtained from a large number of persons in advance; and
a result display section configured to display a rank of the subject regarding each of the habit variables (C2, C3) among the habit variables (C2, C3) of the large number of persons, the rank having been obtained by the computation section, wherein
the health state of the subject is evaluated by obtaining, according to the habit variables (C2, C3), the rank of the subject among the data of the large number of persons, the data having been obtained in advance, where
Ax is a weight coefficient of each Dx, which is derived through the principal component analysis,

$$\text{nutrition balance habit variable } C2 = \Sigma(A2,n2*D2,n2) + \Sigma(A1,n1*D1,n1)$$

(D1,n1 includes one or more of minimal blood pressure, AST, ALT, and blood glucose; n1 is a natural number, $\Sigma$ 1 to n1)
(D2,n2 includes one or more of LDL, HDL, $\gamma$GTP, and neutral fats; n2 is a natural number, $\Sigma$ 1 to n2), and

$$\text{exercise habit variable } C3 = A3(\text{intrinsic coefficient of maximal blood}$$
$$\text{pressure})*D3(\text{maximal blood pressure}) + \Sigma(A1,n1*D1,n1)$$

(n1 is a natural number, $\Sigma$ 1 to n1).

Fig. 1

Fig. 2

## Fig. 3

ENERGY INTAKE | NUTRITION BALANCE | EXECISE

1 DYSLIPIDEMIA
2 HIGH BLOOD PRESSURE
3 LIVER DYSFUNCTION
4 GLUCOSE METABO-LISM DISORDER
5 LOW BLOOD PRESSURE
6 ANEMIA
7 OTHRES
8 NO FINDING

(a)

(NUMBER OF PERSONS:5,000)

ENERGY INTAKE
SCORE DISTRIBUTION

(X 100%)

(b)

NUTRITION BALANCE
SCORE DITRIBUTION

(c)

EXERCISE AMOUNT
SCORE DISTRIBUTION

Fig. 4

EP 3 451 276 A1

Fig. 5

(a)

ENERGY INTAKE HABIT

SMALL ⟵ ENERGY INTAKE GREAT

(b)

NUTRITION BALANCE HABIT

GOOD ⟵ NUTRITION BALANCE UNBALANCE

(c)

EXERCISE HABIT

ENOUGH ⟵ EXERCISE AMOUNT NOT ENOUGH

(a)

ENERGY INTAKE HABIT

(b)

NUTRITION BALANCE HABIT

(c)

EXERCISE HABIT

(d)

Fig. 6

EP 3 451 276 A1

17

(a) ENERGY INTAKE SCORE DISTRIBUTION

(b) NUTRITION BALANCE SCORE DISTRIBUTION

(c) EXERCISE AMOUNT SCORE DISTRIBUTION

Fig. 7

EP 3 451 276 A1

(a) ENERGY INTAKE SCORE DISTRIBUTION

(b) NUTRITION BALANCE SCORE DISTRIBUTION

(c) EXERCISE AMOUNT SCORE DISTRIBUTION

Fig. 8

EP 3 451 276 A1

19

Fig. 9

(a) ENERGY INTAKE SCORE / PREVALANCE(%)
(b) NUTRITION BALANCE SCORE / PREVALANCE(%)
(c) EXERCISE SCORE / PREVALANCE(%)

EP 3 451 276 A1

Fig. 10

**INTERNATIONAL SEARCH REPORT**

| International application No. |
| --- |
| PCT/JP2016/067547 |

| A. CLASSIFICATION OF SUBJECT MATTER |
| --- |
| G06Q50/22(2012.01)i |

According to International Patent Classification (IPC) or to both national classification and IPC

| B. FIELDS SEARCHED |
| --- |
| Minimum documentation searched (classification system followed by classification symbols) |
| G06Q50/22 |

Documentation searched other than minimum documentation to the extent that such documents are included in the fields searched

| | | |
| --- | --- | --- |
| Jitsuyo Shinan Koho | 1922–1996 | Jitsuyo Shinan Toroku Koho 1996–2016 |
| Kokai Jitsuyo Shinan Koho | 1971–2016 | Toroku Jitsuyo Shinan Koho 1994–2016 |

Electronic data base consulted during the international search (name of data base and, where practicable, search terms used)

| C. DOCUMENTS CONSIDERED TO BE RELEVANT | | |
| --- | --- | --- |
| Category* | Citation of document, with indication, where appropriate, of the relevant passages | Relevant to claim No. |
| P,A | JP 2016-91338 A (Kuintesenshia Godo Kaisha), 23 May 2016 (23.05.2016), entire text; all drawings (Family: none) | 1-11 |
| A | JP 5870328 B1 (Kabushiki Kaisha FiNC), 24 February 2016 (24.02.2016), entire text; all drawings (Family: none) | 1-11 |
| A | JP 2010-61389 A (Tokyo Women's Medical University), 18 March 2010 (18.03.2010), entire text; all drawings (Family: none) | 1-11 |

| ☐ Further documents are listed in the continuation of Box C. | ☐ See patent family annex. |
| --- | --- |

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 08 August 2016 (08.08.16) | 16 August 2016 (16.08.16) |

| Name and mailing address of the ISA/ | Authorized officer |
| --- | --- |
| Japan Patent Office 3-4-3,Kasumigaseki,Chiyoda-ku, Tokyo 100-8915,Japan | |
| | Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

• Reference Range for basic tests of new medical checkup, MEGA Study of 1.5 million subjects by the Japan Society of Ningen Dock and the National Federation of Health Insurance Societies. Japan Society of Ningen Dock/National Federation of Health Insurance Societies, April 2014